# EUROPÄISCHE PATENTSCHRIFT

(11) **EP 3 525 746 B1**
(45) Veröffentlichungstag und Bekanntmachung des Hinweises auf die Patenterteilung: **02.12.2020**
(21) Anmeldenummer: 17790959.5
(22) Anmeldetag: 06.10.2017
(51) Int. Cl.: A61H 39/00

(54) **AUFLAGE FÜR EINE UNTERFEDERUNG UND VERFAHREN ZUM SENSORISCHEN TRAINING EINER PERSON**
REST FOR A MATTRESS SUPPORT SYSTEM AND METHOD FOR SENSORY TRAINING OF A PERSON
REVÊTEMENT POUR SOMMIER ET PROCÉDÉ POUR LA RÉÉDUCATION SENSORIELLE D'UNE PERSONNE

(30) Priorität: 14.10.2016 DE 102016012244
(43) Veröffentlichungstag der Anmeldung: 21.08.2019
(73) Patentinhaber: Thomas GmbH + Co. Technik + Innovation KG, 27432 Bremervörde (DE)
(72) Erfinder: JANSEN, Klaus, 21614 Buxtehude (DE)
(74) Vertreter: Möller, Friedrich
(86) Internationale Anmeldenummer: PCT/EP2017/001185
(87) Internationale Veröffentlichungsnummer: WO 2018/068886

(56) Entgegenhaltungen:
- WO-A1-2015/143430
- WO-A2-2014/138504
- DE-A1- 3 537 210
- US-A1- 2014 350 441
- US-A1- 2016 030 281

## Beschreibung

Die Efindung betrifft eine Auflage für eine Unterfederung gemäß Anspruch 1.

WO2015/143430 offenbart eine Auflage für eine Unterfederung für insbesondere eine Matratze eines Schlaf-,Sitz- oder Liegemöbels für ein sensorisches Training einer Person mit einer Vielzahl von individuell oder gruppenweise ansteuerbaren und sensorische Reize auslösenden mikro-stimulierenden Elementen, die auf einer Oberfläche der Auflage angeordnet oder in die Auflage integriert sind und mit einer Steuereinrichtung, die Mittel aufweist zur individuellen oder gruppenweisen und aktiven oder passiven Ansteuerung der mikro-stimulierenden Elemente während des Trainings und zur Erzeugen eines Feedback-Signals auf die sensorischen Reize. WO2015/143430 offenbart nicht, dass die Ansteuerung durch die trainierende Person betätigt wird und dass die mikro-stimulierenden Elemente mit einem Abstand gleich oder unterhalb der Diskriminierungsschwelle des menschlichen Körpers für die Wahrnehmung von Reizen auf oder in der Auflage angeordnet sind, wobei dieser Abstand für verschiedene Körperbereiche der Person variierbar ist und wobei die Steuereinrichtung kabellos oder kabelgebunden mit den mikro-stimulierenden Elementen verbunden ist und ein mobiles Kommunikationsgerät ist, das mit einer entsprechenden Anwendung zur Ansteuerung der mikro-stimulierenden Elemente und mit einem Speicher zum Speichern von Daten ausgestattet ist. WO2014/138504 offenbart ein System zur Ausübung von Kräften auf dem Körper, damit die Schwerkraft, Schwerelosigkeit und Auftrieb simuliert werden kann. US 2014/0350441 offenbart ein System zur vibratorischen neuronalen Stimulation als Hilfe gegen Schmerz unter Verwendung eines vorprogrammierten oder vom Benutzer definierten Musters für die entsprechende Körperregion. US2016/0030281 offenbart ein System mit mehreren Vibratoren für die Stimulation des Körpers.

Bei bekannten Unterfederungen für insbesondere eine Matratze eines Schlaf-, Sitz- oder Liegemöbels zur Behandlung von Erkrankungen wie beispielsweise Demenz, Parkinson, Dekubitus oder Koma dienen Massagemittel dazu, bestimmte Reize bei einer auf der Unterfederung liegenden Person auszulösen. In der Regel wird dazu eine Massagefunktion in der Unterfederung gestartet, welche pauschal, d. h. ohne gewisse Körperregionen zu selektieren, eine physische Kraft auf die Person ausübt.

Andererseits ist es auch bekannt, dass sich einzelne Massageelemente oder Gruppen dieser Elemente ansteuern lassen, um nur bestimmte Körperregionen einer periodischen und mechanischen Kraft auszusetzen. Je nach diagnostizierter Erkrankung können dann verschiedene Körperregionen mit verschiedensten mechanischen Stimulationen behandelt werden. Die Art und Intensität dieser Behandlung wird meist durch die behandelnde Person bestimmt. Oftmals richtet sich diese Behandlung nach Erfahrungswerten, welche bereits bei zu behandelnden Personen mit einem ähnlichen Krankheitsbild angewandt wurden.

Als besonders nachteilig hat sich bei diesem Verfahren erwiesen, dass sie der zu behandelnden Person keinerlei Möglichkeiten bietet, die Behandlung selbst zu steuern bzw. zu kontrollieren. Dadurch gestalten sich die bekannten Verfahren und Methoden als äußerst ineffizient.

Der Erfindung liegt daher die Aufgabe zugrunde, eine Auflage für eine Unterfederung sowie ein Verfahren zum sensorischen Training zu schaffen, mit der ein besonders effiziente Behandlung einer Person möglich ist.

Eine Auflage zur Lösung dieser Aufgabe weist die Merkmale des Anspruchs 1 auf. Demnach sieht die Erfindung eine Auflage für eine Unterfederung für insbesondere eine Matratze eines Schlaf-, Sitz- oder Liegemöbels für ein sensorisches Training einer Person vor. Diese Auflage weist eine Vielzahl von individuell oder gruppenweise ansteuerbaren und sensorische Reize auslösenden, mikro-stimulierenden Elementen auf. Diese Elemente sind auf einer Oberfläche der Auflage angeordnet oder in die Auflage direkt integriert. Erfindungsgemäß weist die Auflage außerdem eine Steuereinrichtung auf, welche Mittel zur individuellen oder gruppenweisen und aktiven oder passiven Ansteuerung der mikro-stimulierenden Elemente durch die Person während des Trainings bereithält. Außerdem dient diese Steuereinrichtung der Erzeugung eines Feedback-Signals auf die sensorischen Reize. Durch diese Auflage, insbesondere durch diese Steuereinrichtung, kann die Person somit nicht nur das Training selbst gestalten bzw. steuern, vielmehr ermöglicht es die Steuereinheit der Person, ein Feedback-Signal auf die sensorischen Reize zu erzeugen. Insbesondere durch dieses Feedback-Signal ist feststellbar, ob die erzeugten sensorischen Reize von der Person wahrnehmbar sind. In Abhängigkeit von diesem Feedback-Signal können die mikro-stimulierenden Elemente durch die Person selbst oder durch die Steuereinrichtung für das weitere Training angesteuert werden. Es ist jedoch erfindungsgemäß auch vorgesehen, dass die mikro-stimulierenden Elemente passiv angesteuert werden. Dies gestaltet sich insbesondere für die Behandlung von komatösen Personen, die nicht aktiv die Steuereinrichtung bedienen können, als vorteilhaft.

Erfindungsgemäß ist es außerdem vorgesehen, dass die mikro-stimulierenden Elemente mit einem Abstand gleich, insbesondere unterhalb, der Diskriminierungsschwelle des menschlichen Körpers für die Wahrnehmung von Reizen auf und/oder in der Auflage angeordnet sind, wobei dieser Abstand für verschiedene Körperbereiche der Person variierbar ist. Insbesondere für die Verbesserung der Körperwahrnehmung bzw. für die Verbesserung der Wahrnehmung des Körperbildes der Person wird die Diskriminierungsschwelle zwischen zwei örtlich versetzten Reizen untersucht. Dabei wird konkret bestimmt, wie genau die Person einen Ort eines Reizes in Bezug auf den eigenen Körper definieren kann. Um den Wert dieser Diskriminierungsschwelle der Person nicht nur bestimmen zu können, sondern vielmehr auch zu verbessern, ist es wesentlich, dass der Abstand der mikro-stimulierenden Elemente sehr gering ist. Die Abstände lassen sich variieren. Somit lassen sich Auflagen individuell für verschiedene Personen mit unterschiedlichen Physiognomien und für verschiedene Körperbereiche erstellen. Es ist beispielsweise denkbar, dass die Elemente über Klettverschlüsse, Knöpfe, Magnete oder dergleichen an verschiedenen Positionen auf oder in bzw. unter der Auflage positionierbar sind. Es ist jedoch auch denkbar, dass die mikro-stimulierenden Elemente fest, d. h. unlösbar, mit der Auflage verbunden sind.

Ein weiteres bevorzugtes Ausführungsbeispiel der vorliegenden Erfindung kann es vorsehen, dass es sich bei den mikro-stimulierenden Elementen um Aktuatoren handelt, die eine Vibration, einen taktilen Impuls oder einen Stoß erzeugen oder um Mittel zur Erzeugung von lokalen und/oder kurzen Hitze- oder Kälteimpulsen, deren Intensität bzw. Temperaturen kontinuierlich veränderbar sind. Somit ist es denkbar, dass durch die Auflage ein sensorischer Impuls oder ein Empfinden generiert wird, deren Intensität solange erhöht bzw. verringert werden kann, bis es von der Person wahrgenommen bzw. nicht mehr wahrgenommen wird. Diese Veränderlichkeit lässt sich über die Steuereinrichtung von der zu behandelnden Person selbst aktiv oder passiv regeln. Somit gestaltet sich die erfindungsgemäße Auflage auch für den außerklinischen Bereich bzw. für die Nutzung zu Hause als besonders geeignet.

Weiter kann es die Erfindung vorteilhafter Weise vorsehen, dass die mikro-stimulierenden Elemente Quellen elektromagnetischer Strahlung, insbesondere punktuelle Lichtquellen, darstellen, deren Wellenlänge und oder Intensität vorzugsweise variierbar sind. Es hat sich gezeigt, dass die Bestrahlung mit Licht einer bestimmten Wellenlänge, insbesondere mehreren Wellenlängen, sich besonders vorteilhaft auf das Wohlbefinden des menschlichen Körpers auswirken kann. Neben der wärmenden Infrarot-Strahlung wird auch die Bestrahlung mit kurzwelligerem Licht, beispielsweise aus dem sichtbaren Sonnenspektrum, als sehr angenehm empfunden. Diese Bestrahlung kann durch jede Person individuell, sowohl in der Wellenlänge als auch in der Intensität, eingestellt werden. Dieses Variieren der Wellenlänge bzw. der Intensität kann beispielsweise mittels Filter und einer Weißlichtquelle realisiert werden.

Weiterhin es es erfindungsgemäß vorgesehen, dass die Steuereinrichtung kabellos mit dem mikro-stimulierenden Elementen verbunden ist, dass es sich bei der Steuereinrichtung um ein mobiles Kommunikationsgerät handelt mit einer entsprechenden Anwendung zur Ansteuerung der mikro-stimulierenden Elemente und mit einem Speicher zum Speichern von Daten. Somit kann es sich bei der Steuereinrichtung beispielsweise um ein Tablet oder um ein Smartphone handeln, auf dem eine passende *App* installiert ist. Dadurch lassen sich die Daten der Behandlung bzw. des Trainings automatisch auf verschiedene Geräte übertragen, sodass die behandelnde Person stets über den Fortgang der Behandlung informiert ist. Außerdem gestaltet es sich als besonders vorteilhaft, dass die Daten permanent und fortwährend gespeichert werden. So lassen sich die Daten von einer vorherigen Trainingseinheit aufrufen und mit den aktuellen Trainingsdaten vergleichen. Durch diesen Vergleich der Werte lässt sich der Trainingsfortschritt bewerten, wodurch das weitere Training bzw. die weitere Behandlung individuell auf die zu behandelnde Person angepasst und gestaltet werden kann. Diesen Trainingsfortschritt kann sich die Person auch direkt auf dem Tablet oder dem Smartphone anzeigen lassen, um selbst den eigenen Fortschritt des Trainings beurteilen zu können. Daher eignet sich die Auflage nicht nur als klinisches Therapieinstrument, sondern vielmehr auch als Trainingsgerät für privaten Gebrauch.

Insbesondere kann es die Erfindung vorsehen, dass die mikro-stimulierenden Elemente derart dimensioniert sind, insbesondere einen Durchmesser von 1 bis 10 mm, vorzugsweise 2 bis 5 mm aufweisen, dass sie im inaktiven Zustand von der Person auf der Auflage nicht wahrnehmbar sind. Somit lässt sich die Auflage auch als konventionelle Unterfederung für eine Matratze verwenden. Durch die Dimensionierung der mikro-stimulierenden Elemente wirken diese im inaktiven Zustand nicht stimulierend, da sie von der Person nicht wahrnehmbar sind. Erst durch die Aktivierung der mikro-stimulierenden Elemente durch die Steuereinrichtung sind die Elemente durch die hervorrufenden Reize wahrnehmbar.

Es ist ein Verfahren zum sensorischen Training einer Person mit einer Auflage für eine Unterfederung für insbesondere eine Matratze eines Schlaf-, Sitz- oder Liegemöbels, vorgesehen, wobei die Auflage eine Vielzahl von individuell oder gruppenweise ansteuerbaren und sensorische Reize auslösenden mikro-stimulierenden Elemente aufweist. Diese Elemente werden durch eine Steuereinrichtung individuell oder gruppenweise von der Person aktiv oder passiv gesteuert. Zur passiven Steuerung wird ein Feedback-Signal verwendet, welches von der Steuereinrichtung anhand der Reaktion der Person auf die Reize erzeugt wird. Durch das Erzeugen dieses Feedback-Signals kann die Person unmittelbar Einfluss nehmen auf das Training. Gleichzeitig erhält eine behandelnde Person eine direkte Aussage darüber, wie der Trainingszustand der Person bzw. der Trainingsfortschritt der Person ist. Somit lässt sich durch dieses Verfahren die Effizienz des Trainings bzw. der Behandlung verbessern.

Es ist auch vorgesehen, dass zur Erzeugung eines Reizschemas oder zur Modifikation oder Stabilisierung eines Körperbildes der Person die mikro-stimulierenden Elemente variabel angesteuert werden, wobei insbesondere die mikro-stimulierenden Elemente derart aktiviert werden, dass ihre Intensität und der Abstand zu einem nächsten aktiven mikro-stimulierenden Element in Abhängigkeit von dem Feedback-Signal der Person variiert werden. Durch diese Abhängigkeit der Ansteuerung der Elemente von den erfassten Reizen kann ein besonders genaues Körperbild der Person erstellt werden. Des Weiteren lässt sich während des Trainings eine eventuelle Änderung dieses Körperbilds untersuchen. Je nach Entwicklung des Reizschemas und/oder des Körperbildes kann die Aktivierung der mikro-stimulierenden Elemente verändert werden. Dadurch erhält sowohl die zu behandelnde Person als auch die behandelnde Person die Möglichkeit, das Training sowie den Trainingsfortschritt aktiv und somit sehr effizient zu steuern.

Es ist auch vorgesehen, dass die mikro-stimulierenden Elemente derart angesteuert werden, dass der erzeugte sensorische Reiz gerade noch von der Person wahrnehmbar bzw. dass gerade noch zwischen zwei örtlich getrennten Reizen differenziert werden kann, wobei in einem folgenden Trainingsschritt diese Reizschwelle durch die Steuereinrichtung erneut angesteuert wird und die Feedback-Signale der Person verglichen werden. Durch diese Untersuchung der Diskriminierungsschwelle der Person kann das Körperbild der Person vermessen werden und als Grundlage für ein effizienteres Training benutzt werden. Dieses Verfahren hat sich als besonders hilfreich erwiesen für die Behandlung von Parkinson, Demenz, Dekubitus oder Komapatienten.

Es ist auch vorgesehen, dass die Feedback-Signale der Person auf die Reize der mikro-stimulierenden Elemente aktiv durch die Person oder passiv durch Sensoren erzeugt werden. Die Person kann aktiv über die Steuereinheit direkt ein Feedback-Signal erzeugen, mit dem sie ausdrückt, dass sie den Reiz gar nicht, schlecht oder gut wahrgenommen hat. Außerdem kann die Person anhand der Steuereinrichtung benennen, an welcher Position der oder die Reize wahrgenommen wurden. Gleichermaßen können die Reize bei beispielsweise einer komatösen Person durch Sensoren direkt am Körper der Person nachgewiesen werden. Diese Sensoren wiederrum erzeugen über die Steuereinrichtung ein Feedback-Signal, welches wiederrum dazu dient, die weitere Behandlung oder das weitere Training individuell auf die Person abzustimmen.

Ein Trainingsbeispiel kann es vorsehen, dass für gezielte Trainingszwecke mikro-stimulierende Elemente für ausgewählte Körperregionen oder -teile aktiviert werden, um aktivierende Impulse auf innere Organe geben zu können oder um eine gezielte Veränderung der Repräsentation eines oder mehrerer Körperteile im neuronalen System der Person zu erreichen. Dadurch lässt sich für nahezu jede Erkrankung das Training individuell für die Person einstellen, um ein maximal effizientes Training zu realisieren.

Weiter kann das Training vorsehen, dass die aktivierenden Impulse so gesteuert werden, dass Stoffwechselprozesse oder die Durchblutung in den stimulierenden Bereichen oder auch ganzkörperlich modifiziert werden. Durch diese punktuelle Modifikation der Durchblutung und/oder des Stoffwechselprozesses lassen sich gezielt traumatisierte Körperbereiche behandeln. Andererseits lassen sich durch das erfindungsgemäße Verfahren bzw. durch die Erfindung nicht nur therapeutische Behandlungen sondern auch Behandlungen, die das allgemeine Wohlbefinden verbessern, also der *Wellness* dienen, durchführen.

Ein bevorzugtes Ausführungsbeispiel der vorliegenden Erfindung wird nachfolgend anhand der Zeichnung näher erläutert. In dieser zeigen:
- Fig. 1: eine perspektivische Darstellung einer Auflage mit einer Person, und
- Fig. 2: eine Sicht auf die in Fig. 1 dargestellte Person auf der Auflage,
Bei der hier beispielhaft dargestellten erfindungsgemäßen Auflage 10 handelt es sich um eine Auflage für eine Unterfederung für eine Matratze eines nicht dargestellten Schlaf- oder Liegemöbels. Diese in den Figuren dargestellte Auflage 10 ist quaderförmig ausgebildet und derart bemessen, dass eine durchschnittlich große, erwachsene Person 13 auf dieser liegend Platz findet. Gleichzeitig soll darauf hingewiesen werden, dass die Auflage erfindungsgemäß auch für Sitzmöbel oder dergleichen benutzt werden kann.

Die hier dargestellte Auflage 10 dient in erster Linie einem sensorischen Training einer zu behandelnden Person 13. Darüber hinaus kann die Auflage 10 jedoch auch für Massage- oder sonstige Wellness-Anwendungen verwendet werden. Für dieses Training bzw. für diese Anwendung weist die in den Figuren dargestellte Auflage 10 auf ihrer Oberfläche 11 eine Vielzahl von mikro-stimulierenden Elementen 12 auf. Diese mikro-stimulierenden Elemente 12 können beliebig über die gesamte Oberfläche 11 der Auflage 10 verteilt sein. Gleichermaßen ist es denkbar, dass die mikro-stimulierenden Elemente 12 in die Auflage 10 integriert oder auf einer Unterseite der Auflage 10 angeordnet sind.

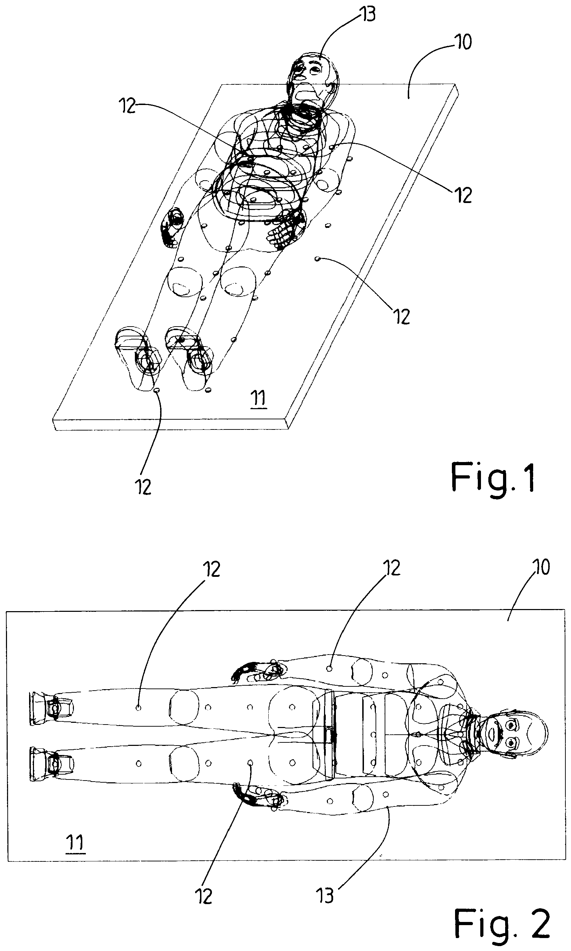

Für das Verfahren sind die mikro-stimulierenden Elemente auf der Oberfläche 11 der Auflage 10 derart angeordnet, dass wenigstens nahezu alle Körperbereiche der zu behandelnden Person 13 erreicht werden können. In den Fig. 1, 2 ist eine schematisierte Person 13 auf der Auflage 10 liegend dargestellt. Insbesondere der Fig. 2 ist zu entnehmen, dass die mikro-stimulierenden Elemente 12 genau unter allen Gliedmaßen bzw. dem Körper der Person 13 angeordnet sind. D. h. dass sowohl unter den Füßen, unter den Beinen, als auch unter den Armen und den Händen usw. mikrostimulierende Elemente 12 angeordnet sind.

Um den unterschiedlichen Körpergrößen und Formen gerecht zu werden, kann es vorgesehen sein, dass die mikro-stimulierenden Elemente 12 lösbar auf der Oberfläche 11, beispielsweise durch einen Klettverschluss, befestigbar sind. Alternativ ist es jedoch auch vorgesehen, dass die gesamte Oberfläche 11 der Auflage 10 mit einem engen Raster der mikro-stimulierenden Elemente 12 abgedeckt ist, sodass unabhängig von der Körperform der Person 13 alle Körperteile durch die mikro-stimulierenden Elemente 12 erfassbar sind. Darüber hinaus kann es vorgesehen sein, dass bestimmte Körperpartien wie beispielweise einem Oberschenkel oder den Füßen weniger bzw. mehr und anderen Körperbereichen wie dem Rücken oder dem Kopf mehr bzw. weniger mikro-stimulierenden Elemente 12 zugeordnet sind. Außerdem kann der Abstand zwischen benachbarten mikro-stimulierenden Elementen 12 in Abhängigkeit von der Körperregion der Person 13 variieren. So ist es beispielsweise denkbar, dass der vermeintlichen Rückenposition der zu behandelnden Person 13 eine höhere Flächendichte an mikro-stimulierenden Elementen 12 zugeordnet ist als den Bereichen der Unterschenkel.
Bei den mikro-stimulierenden Elementen 12 kann es sich um Aktuatoren handeln, die eine Vibration, einen taktilen Impuls oder einen mechanischen Stoß erzeugen oder um Mittel zur Erzeugung von lokalen und/oder kurzen Hitze- oder Kälteimpulsen, deren Intensitäten bzw. Temperaturen kontinuierlich veränderbar sind. Außerdem kann es sich bei den mikro-stimulierenden Elementen 12 um Quellen elektromagnetischer Strahlung wie beispielsweise Lichtquellen, deren Wellenlänge und/oder Intensität variabel sind, handeln. Durch diese mikro-stimulierenden Elemente 12 oder einer Kombination dieser lässt sich je nach Krankheitsbild und Art der Behandlung bzw. Anwendung die zu behandelnde Person 13 entsprechend behandeln. So ist es beispielsweise denkbar, dass im Lendenwirbelbereich punktuell Hitze erzeugt wird, während das linke Bein der Person 13 durch periodische, mechanische Impulse oder Stromstöße stimuliert wird.

Die Energieversorgung der mikrostimulierenden Elemente 12 kann kabelgebunden oder kabellos erfolgen. Demnach ist es beispielsweise denkbar, dass die mikro-stimulierenden Elemente 12 eine Batterie aufweisen. Gleichermaßen ist es denkbar, dass die mikro-stimulierenden Elemente 12 ihre Energie über elektromagnetische Strahlung erhalten, oder dass die Elemente 12 über ein Kabel mit einer entsprechenden Energiequelle verbunden sind.

Je nach Art der Elemente 12 und deren Anwendung können sich diese in ihrer Größe unterscheiden. Erfindungsgemäß ist es vorgesehen, dass die Elemente 12 einen Durchmesser von 1 bis 10mm, vorzugsweise 2 bis 5mm, aufweisen. Die Erfindung zieht es vor, dass die mikro-stimulierenden Elemente 12 derart bemessen und angeordnet sind dass sie im inaktiven Zustand von der Person 13 nicht wahrgenommen werden.

Der Übersicht halber sind in den Fig. 1 und 2 jeweils nur einige der dargestellten kreisförmigen mikro-stimulierenden Elemente 12 mit einem Bezugszeichen versehen.

Zur Steuerung der mikro-stimulierenden Elemente 12 ist der Auflage 10 eine nicht dargestellte Steuereinrichtung zugeordnet. Diese Steuereinrichtung kann durch ein Kabel oder kabellos mit allen mikro-stimulierenden Elementen 12 gleichermaßen kommunizieren. Durch diese Steuereinrichtung lassen sich einzelne mikro-stimulierende Elemente 12, Gruppen dieser oder alle Elemente 12 ansteuern bzw. regeln. Für eine besonders ergonomische Handhabung der Steuereinrichtung ist es vorgesehen, dass diese beispielsweise als Tablet-PC, Smart-Phone oder dergleichen ausgebildet ist. Über diesen Tablet-PC kann die behandelnde Person oder die zu behandelnde Person 13, vorzugsweise über eine *App,* die mikro-stimulierenden Elemente 12 steuern. So ist es denkbar, dass auf der Steuereinrichtung personenspezifische und krankheitsbildspezifische Programme gespeichert sind, mit denen die Person 13 selbstständig das sensorische Training bzw. die Behandlung durchführen kann. Ganz wesentlich dabei ist, dass sich über die Steuereinheit das Training nicht nur starten und steuern lässt, sondern auch ein Feedback der Person 13 eingeben lässt. Auf diese Weise kann die zu behandelnde Person 13 selbstständig eingeben, in welcher Körperposition sie einen Reiz wahrnimmt. In Abhängigkeit von dieser Wahrnehmung kann die Person 13 selbstständig die Intensität des Reizes punktuell oder global erhöhen oder soweit verringern, bis ein gewisser Wahrnehmungsschwellenwert unterschritten wird.

Darüber hinaus kann die Person 13 über die Steuereinrichtung ermitteln, inwieweit sie voneinander beabstandete mikro-stimulierende Elemente 12 noch voneinander trennen kann. Durch die Ansteuerung mehrerer benachbarter mikro-stimulierender Elemente 12 kann der Abstand bzw. die sogenannte Diskriminierungsschwelle zwischen zwei noch voneinander trennbaren mikro-stimulierenden Elementen 12 ausgemessen und verbessert werden. Durch das permanente, vorzugsweise kontinuierliche, Speichern der Feedback-Signale der Person 13 können ältere Trainingsdaten abgerufen und mit aktuellen Trainingsdaten verglichen werden, um so den Trainingsfortschritt beurteilen zu können. In Abhängigkeit von diesem Trainingsfortschritt kann dann das weitere Programm für die Behandlung individuell für die Person 13 erstellt werden.

Durch diese gezielte punktuelle oder globale Stimulierung der Person 13 und deren Feedback auf die einzelnen Reize lässt sich ein Körperbild der gesamten Person 13 erstellen, welches der behandelnden Person Aufschluss über die Erkrankung bzw. über den Fortschritt der Behandlung bietet.

Neben der zuvor beschriebenen aktiven Ansteuerung der mikro-stimulierenden Elemente 12 durch die Person 13 lassen sich diese auch passiv durch die Person 13 aktivieren. Diese passive Ansteuerung ist insbesondere für die Behandlung von Komapatienten vorteilhaft. Für diese passive Ansteuerung sind an der Person 13 entsprechende Sensoren anzubringen, welche nachweisen können, dass die Person 13 einen Reiz durch die mikro-stimulierenden Elemente 12 erfahren hat. Durch die von den Sensoren ermittelten Informationen lassen sich wiederrum weitere Signale für die Ansteuerung der mikro-stimulierenden Elemente 12 erzeugen, sodass auch eine im Koma liegende Person 13 gezielt durch die in die Auflage 10 integrierten Elemente 12 behandelt bzw. trainiert werden kann.

Durch die mikro-stimulierenden Elemente 12 lassen sich sowohl Muskeln stimulieren als auch Stoffwechselprozesse. Außerdem lässt sich die Durchblutung anregen. Darüber hinaus werden Reize generiert, die dazu dienen, ausgewählte Körperregionen oder Teile zu aktivieren und aktivierende Impulse auf innere Organe zu erzeugen. Dadurch können gezielte Veränderungen der Repräsentation eines oder mehrerer Körperteile im neuronalen System der Person 13 erzeugt werden. Durch diese Veränderungen können eine Vielzahl von Erkrankungen personenspezifisch und somit effizient behandelt werden.

### Bezugszeichenliste

- 10: Auflage
- 11: Oberfläche
- 12: mikro-stimulierende Elemente
- 13: Person

## Patentansprüche

1. Auflage (10) für eine Unterfederung für insbesondere eine Matratze eines Schlaf-, Sitz- oder Liegemöbels für ein sensorisches Training einer Person (13) mit einer Vielzahl von individuell oder gruppenweise ansteuerbaren und sensorische Reize auslösenden mikro-stimulierenden Elementen (12), die auf einer Oberfläche (11) der Auflage (10) angeordnet oder in die Auflage (10) integriert sind und mit einer Steuereinrichtung, die Mittel aufweist zur individuellen oder gruppenweisen und aktiven Ansteuerung der mikro-stimulierenden Elemente (12) durch die Person (13) oder passiven Ansteuerung durch die Steuereinrichtung während des Trainings und zur Erzeugen eines Feedback-Signals auf die sensorischen Reize, **gekennzeichnet dadurch, dass** die mikro-stimulierenden Elemente (12) mit einem Abstand gleich oder unterhalb der Diskriminierungsschwelle des menschlichen Körpers für die Wahrnehmung von Reizen auf oder in der Auflage (10) angeordnet sind, wobei dieser Abstand für verschiedene Körperbereiche der Person (13) variierbar ist und wobei die Steuereinrichtung kabellos oder kabelgebunden mit den mikro-stimulierenden Elementen (12) verbunden ist und ein mobiles Kommunikationsgerät ist, das mit einer entsprechenden Anwendung zur Ansteuerung der mikro-stimulierenden Elemente (12) und mit einem Speicher zum Speichern von Daten ausgestattet ist.

2. Auflage (10) nach Anspruch 1, **dadurch gekennzeichnet, dass** es sich bei den mikro-stimulierenden Elementen (12) um Aktuatoren handelt die eine Vibration, einen taktilen Impuls oder Stoß erzeugen oder um Mittel zur Erzeugung von lokalen und/oder kurzen Hitze- oder Kälteimpulsen, deren Intensitäten bzw. Temperaturen kontinuierlich veränderbar sind.

3. Auflage (10) nach Anspruch 1 oder 2, **dadurch gekennzeichnet, dass** die mikrostimulierenden Elemente (12) Quellen elektromagnetischer Strahlung, insbesondere punktuelle Lichtquellen, darstellen, deren Wellenlänge und/oder Intensität vorzugsweise variierbar sind.

4. Auflage (10) nach einem der vorhergehenden Ansprüche, **dadurch gekennzeichnet, dass** die mikro-stimulierenden Elemente (12) lösbar mit der Auflage (10) verbindbar sind, insbesondere dass sich die mikro-stimulierenden Elementen (12) auf eine beliebige Art und Weise der Auflage (10) zuordnen lassen.

5. Auflage (10) nach einem der vorhergehenden Ansprüche, **dadurch gekennzeichnet, dass** die mikro-stimulierenden Elemente (12) derart dimensioniert sind, insbesondere einen Durchmesser von 1 bis 10 mm, vorzugsweise 2 bis 5 mm aufweisen, dass sie im inaktiven Zustand von der Person (13) auf der Auflage nicht wahrnehmbar sind.

## Claims

1. A rest (10) for a mattress support system, in particular for a mattress of an item of furniture for sleeping on, sitting on or lying on, for sensory training of a person (13), with a multiplicity of micro-stimulating elements (12) which can be actuated individually or in groups and trigger sensory stimuli and which are arranged on a surface (11) of the rest (10) or are integrated in the rest (10), and with a control device having means for active actuation of the micro-stimulating elements (12), individually or in groups, by the person (13), or passive actuation by the control device, during the training and for generating a feedback signal in response to the sensory stimuli, **characterized in that** the micro-stimulating elements (12) are arranged on or in the rest (10) at a spacing equal or below the discrimination threshold of the human body for the perception of stimuli, this spacing being variable for different body regions of the person (13) and wherein the control device is connected wirelessly or by wire to the micro-stimulating elements (12) and is a mobile communication appliance with a corresponding application for actuating the micro-stimulating elements (12) and with a memory for storing data.

2. The rest (10) as claimed in claim 1, **characterized in that** the micro-stimulating elements (12) are actuators which generate a vibration, a tactile impulse or jolt, or are means for generating local and/or short heat or cold impulses, of which the intensities and/or temperatures are continuously modifiable.

3. The rest (10) as claimed in claim 1 or 2, **characterized in that** the micro-stimulating elements (12) constitute sources of electromagnetic radiation, in particular punctiform light sources, of which the wavelength and/or intensity are preferably variable.

4. The rest (10) as claimed in one of the preceding claims, **characterized in that** the micro-stimulating elements (12) are releasably connectable to the rest (10), in particular **in that** the micro-stimulating elements (12) can be assigned in any desired manner to the rest (10).

5. The rest (10) as claimed in one of the preceding claims, **characterized in that** the micro-stimulating elements (12) are dimensioned, in particular with a diameter of 1 to 10 mm, preferably of 2 to 5 mm, in such a way that, in the inactive state, they are not perceptible to the person (13) on the rest.

## Revendications

1. Support (10) destiné à un sommier à ressorts destiné en particulier à un matelas d'un meuble destiné à dormir, s'asseoir ou se coucher destiné à l'entraînement sensoriel d'une personne (13), ledit support comprenant une multitude d'éléments de micro-stimulation (12), déclenchant des stimuli sensoriels et commandables individuellement ou en groupes, qui sont disposés sur une surface (11) du support (10) ou qui sont intégrés dans le support (10), et un dispositif de commande qui comporte des moyens de commande active, individuelle et en groupe, des éléments de micro-stimulation (12) par le biais de la personne (13) ou de commande passive par le biais du dispositif de commande pendant l'entraînement et de génération d'un signal de rétroaction en réponse aux stimuli sensoriels,
**caractérisé en ce que**
les éléments de micro-stimulation (12) étant disposés à une distance inférieure ou égale au seuil de discrimination du corps humain pour la perception de stimuli sur ou dans le support (10), cette distance étant variable pour différentes zones du corps de la personne (13) et le dispositif de commande étant relié avec ou sans fil aux éléments de micro-stimulation (12) et étant un appareil de communication mobile qui est équipé d'une application correspondante destinée à commander les éléments de micro-stimulation (12) et d'une mémoire destinée à mémoriser des données.

2. Support (10) selon la revendication 1, **caractérisé en ce que** les éléments de micro-stimulation (12) sont des actionneurs, qui génèrent une vibration, une impulsion tactile ou un choc, ou un moyen de génération d'impulsions chaudes ou froides locales et/ou courtes dont les intensités ou températures peuvent être modifiées en continu.

3. Support (10) selon la revendication 1 ou 2, **caractérisé en ce que** les éléments de micro-stimulation (12) représentent des sources de rayonnement électromagnétique, notamment des sources de lumière ponctuelles dont la longueur d'onde et/ou l'intensité sont de préférence variables.

4. Support (10) selon l'une des revendications précédentes, **caractérisé en ce que** les éléments de micro-stimulation (12) peuvent être reliés de manière amovible au support (10), en particulier **en ce que** les éléments de micro-stimulation (12) peuvent être associés d'une manière quelconque au support (10).

5. Support (10) selon l'une des revendications précédentes, **caractérisé en ce que** les éléments de micro-stimulation (12) sont dimensionnés, notamment ont un diamètre de 1 à 10 mm, de préférence de 2 à 5 mm, de manière à ne pas être perceptibles lorsque la personne (13) est inactive sur le support.
